# EUROPEAN PATENT SPECIFICATION

(11) **EP 3 740 762 B1**
(45) Date of publication and mention of the grant of the patent: **31.07.2024**
(21) Application number: 19703245.1
(22) Date of filing: 17.01.2019
(51) Int. Cl.: G01N 33/74

(54) **BIOMARKER PREDICTING CORONARY ARTERY DISEASE**
BIOMARKER ZUR VORAUSSAGE VON KORONARARTERIENERKRANKUNG
BIOMARQUEUR DE PRÉDICTION D'UNE MALADIE DE L'ARTERE CORONAIRE

(30) Priority: 17.01.2018 DE 102018100968
(43) Date of publication of application: 25.11.2020
(73) Proprietor: Immundiagnostik AG, 64625 Bensheim (DE)
(72) Inventor: ARMBRUSTER, Franz Paul, 64270 Bobenheim-Roxheim (DE); DSCHIETZIG, Thomas B, 12555 Berlin (DE)
(74) Representative: Benedum, Ulrich Max
(86) International application number: PCT/EP2019/051194
(87) International publication number: WO 2019/141791

(56) References cited:
- EP-A1- 2 631 247
- EP-A1- 2 775 306
- F. CARLSTEDT ET AL: "Serum levels of parathyroid hormone are related to the mortality and severity of illness in patients in the emergency department", EUROPEAN JOURNAL OF CLINICAL INVESTIGATION, vol. 27, no. 12, 6 November 1996 (1996-11-06), GB, pages 977 - 981, XP055565945, ISSN: 0014-2972, DOI: 10.1046/j.1365-2362.1997.2310778.x
- BERTHOLD HOCHER ET AL: "Measuring Parathyroid Hormone (PTH) in Patients with Oxidative Stress - Do We Need a Fourth Generation Parathyroid Hormone Assay?", PLOS ONE, vol. 7, no. 7, 6 July 2012 (2012-07-06), pages e40242, XP055213564, DOI: 10.1371/journal.pone.0040242
- BERTHOLD HOCHER: "Non-oxidized PTH (n-oxPTH) is associated with cardiovascular events and all-cause mortality in patients with secondary hyperparathyroidism undergoing hemodialysis who participated in the EVOLVE trial", AMERICAN SOCIETY OF NEPHROLOGY KIDNEY WEEK 2014, PHILADELPHIA, 1 November 2014 (2014-11-01), XP055566239
- BERTHOLD HOCHER ET AL: "Why Current PTH Assays Mislead Clinical Decision Making in Patients with Secondary Hyperparathyroidism", NEPHRON JOURNALS, vol. 136, no. 2, 1 January 2017 (2017-01-01), CH, pages 137 - 142, XP055566041, ISSN: 1660-8151, DOI: 10.1159/000455289

## Description

### FIELD OF THE INVENTION

The invention relates to analysing of bodily fluids and in particular to methods of determining the parathyroid horrmone concentration in plasma samples of patients.

### BACKGROUND OF THE INVENTION

Excess mortality in patients with chronic kidney disease (CKD) is an important unsolved problem. Annual mortality of patients with chronic kidney disease stage 5 is about 10 to 20%. The parathyroid hormone (PTH) seems being a factor responsible for the excess mortality in patients requiring hemodialysis as a recent study demonstrated a J-shaped association between PTH and mortality. Consequently, PTH has been described being a uremic toxin with multiple systemic effects including bone disorders (renal osteodystrophy), myopathy, neurologic abnormalities, anemia, pruritus, and cardiomyopathy.

Hyperparathyroidism is common in CKD patients and results in significant morbidity and mortality if left untreated. Low as well as high PTH levels measured by current PTH assays are associated with a progression of cardiovascular diseases and substantially increased all-cause mortality in patients on hemodialysis (Floege J. et al, ARO Investigators. Serum iPTH, calcium and phosphate, and the risk of mortality in a European haemodialysis population. Nephrol Dial Transplant.2011;26:1948-1955; Torres PA et al, Calcium-sensing receptor, calcimimetics, and cardiovascular calcifications in chronic kidney disease. Kidney Int. 2012;82:19-25; Souberbielle JC et al. in Parathyroid hormone measurement in CKD. Kidney Int. 2010;77:93-100). Elevated PTH levels have been associated with increased mortality but standard PTH cut-off values have not been sufficiently tested (Melamed ML et al, Third-generation parathyroid hormone assays and all-cause mortality in incident dialysis patients: the CHOICE study, Nephrol Dial Transplant, 2008; 23(5):1650-8). Thus, guidelines have been established aiming to keep PTH in concentrations associated with the lowest morbidity and highest survival. The Kidney Disease Outcomes Quality Initiative (KDOQI) guidelines recommend measuring regularly PTH concentrations of patients with chronic kidney disease (CKD) and adjusting the patients' medication (e.g. vitamin D, phosphate binders, calcimimetics) such that plasma PTH levels are kept within a target range in accordance with the stage of CKD (e.g., 150 to 300 nglL in patients with CKD stage 5). However, there has been no way of reliably predicting coronary disease, cardiovascular mortality or myocardial infarction in CKD patients or other patients. As mentioned PTH is routinely measured in patients on dialysis but owing to significant variations between contemporary assays, reference ranges are highly controversial. Notwithstanding, PTH has been established as a cardiovascular risk marker, notably in relation to artheriosclerosis.

Further technical background thereto can be found in WO02082092A1, EP2631247A1, EP0349545, US 6,030,790, EP 0451867, WO0144818, WO03003986, WO0042437, WO02082092. The huge scientific literature comprises:
GOLDSMITH DJA et al, The case for routine parathyroid hormone monitoring, CLIN J AM SOC NEPHROL, (2013)_8:319 - 320;
HOCHER B et al., Measuring parathyroid hormone (PTH) in patients with oxidative stress--do we need a fourth generation parathyroid hormone assay?, PLOS (2012) 7, page E40242;
HOCHER B et al., Non-oxidized PTH (n-oxPTH) is associated with cardiovascular events and all-cause mortality in patients with secondary hyperparathyroidism undergoing hemodialysis who participated in the EVOLVE trial", AMERICAN SOCIETY OF NEPHROLOGY KIDNEY WEEK 2014, PHILADELPHIA *;*
HOCHER B et al., Why Current PTH Assays Mislead Clinical Decision Making in Patients with Secondary Hyperparathyroidism, IVEPHROIV JOURNALS, vol. 136, no. 2, pages 137-142*;*
Garrett G. et al, Parathyroid hormone measurements, guidelines statements and clinical treatments: a real-world cautionary tale, ANNALS OF CLINICAL BIOCHEMISTRY 2012(49):4-6;
Raggi P et al, The ADVANCE study: a randomized study to evaluate the effects of cinacalcet plus low-dose vitamin D on vascular calcification in patients on hemodialysis, NEPHROLOGY DIALYSIS TRANSPLANTATION (2011) 26(4):1327 - 1339;
Fraser WD, Hyperparathyroidism, LANCET (2009) 374:145F;
John MR ET AL., J. CLIN. ENDOCRINOL. METAB. (1999) 84:4287-4290;
Brossard JH et at., Influence of glomerular filtration rate on non-(1-84) parathyroid hormone (PTH) detected by intact PTH assays, CLIN CHEM. (2000) 46:697-703;
SCHMIDT-GAYK et al., OSTEOLOGlE FORUM, (1999) 5:48-58;
GAiCERAN T et al., Absence of biological effects of oxidized parathyroid hormone-(1-34) in dogs and rats, ENDOCRINOLOGY, (1984), 115:2375 - 2378;
HORIUCHI N, Effects of oxidation of human parathyroid hormone on its biological activity in continuously infused, thyroparathyroidectomized rats, J BONE MINER RES., (1988) 3:353-358;
MARTIN-MATEO MC et al., REN FAIL, (1999) 21:55 - 167;
HASSELWANDER O et al., FREE RADIC RES, (1998) 29:1 - 11;
ZOCCALI C et al., NEPHROL DIAL TRANSPLANT, (2000) 15, no. 2;
CANAUD B et al., BLOOD PURIF, (1999) 17:99 - 106;
ALEXIEWICZ L M et al., J AM SOC NEPHROL, (1990) 1:236 - 244;
Zull JE et al., Effect of methionine oxidation and deletion of amino-terminal residues on the conformation of parathyroid hormone. Circular dichroism studies, J BIOL CHEM. (1990) 265:5671 - 5676;
ZULL J E et al., J BIOL CHEM, (1990) 265:5671 - 5676;
SUVA et al., SCIENCE, (1987) 237:893FF;
LEPAGE R. et al., CLIN. CHEM. (1998) 44:805 - 809;
PITTS T O et al., MINER ELECTROLYTE METAB, (1988) 15:267 - 275;
HORIUCHI N, J BONE MINER RES, (1988) 3:353 - 358;
FRELINGER A L et al., ARCH BIOCHEM BIOPHYS, (1986) 244:641 - 649;
GALCERANT et al., ENDOCRINOLOGY, (1984) 115:2375 - 2378;
Goltzmann D et al., Discordant disappearance of bioactive and immunoreactive parathyroid hormone after parathyroidectomy, J CLIN ENDOCRINOL METAB. (1984) 58(1):70-75;
FRELINGER A L et al., J BIOL CHEM, (1984) 259:5507 - 5513
O'RiORDAN JLH et al., J ENDOCR. (1974) 63:117 - 124;
LOGUE FC et al., ANN CLIN BIOCHEM. (1991) 28:1 60 - 166;
LOGUE F C, J IMMUN METH. (1991) 39, page 159
NEUMAN WF et al., The metabolism of labeled parathyroid hormone. V. Collected biological studies, CALCIF TISSUE RES. (1975) 18:271 - 287;
HRUSKA KA et al., Peripheral metabolism of intact parathyroid hormone. Role of liver and kidney and the effect of chronic renal failure, J CLIN INVEST., (1981) 67:885 - 892;
Berson SA et al, PNAS USA (1963) 49:613 - 617;

The present applicants have developed an assay for determining individually and simultaneously n-oxPTH and oxPTH, say of non-oxidized bioactive PTH (n-oxPTH) and for oxidized inactive PTH (oxPTH) only. Non-oxPTH results differ from intact PTH (iPTH) obtained with conventional second- and third-generation PTH assays which results reflect a varying blend of bioactivity and oxidative stress: In the EVOLVE study, only non-oxPTH is predictive of the primary endpoint.

However, there is still no method for determining the risk of a myocardial infarction (ICD-10 - 121) in accordance with the *International Classification of Diseases and Related Health Problems* (**ICD**-10) in CKD and patients highly at risk of developing coronary disease. In other words, there is also no biomarker available for long-term monitoring the risk of a myocardial infarction in patients subjected to CKD and other therapy. Myocardial infarction, commonly known as a heart attack, occurs when blood flow decreases or stops to a part of the heart, causing damage to the heart muscle. It should therefore be distinguished from atheriosclerosis, and other cardiovascular diseases.

The prior art therefore represents a problem and it is an object of the present application to provide a predictive biomarker for the risk of a myocardial infarction in CKD and patients at risk of developing a coronary diseases or myocardial infarction and for monitoring respective therapies.

### SUMMARY OF THE INVENTION

This object is achieved by a method of determining the health risk for myocardial infartion in patients subjected to coronary angiography, wherein the concentration of parathyroid hormone is determined *in vitro* in a sample of serum or plasma of said patient and wherein a respective sample of serum or plasma of said patient is contacted with beads specifically binding oxidized parathyroid hormone, and correlating the determined levels of oxidized and non-oxidized parathyroid hormone to the risk for myocardial infarction. According to the invention, the concentration of parathyroid hormone is determined *in vitro* in a first sample of serum or plasma of said patient and in a second sample of serum or plasma of said patient after contacting said sample with beads binding oxidized parathyroid hormone, and correlating the determined levels of parathyroid hormone in said first and second sample to the risk of developing a myocardial infarction by said patient.

In a preferred embodiment, the level of parathyroid hormone is determined conventionally with a second or third-generation PTH immunoassay prior and after removal of the oxidized parathyroid hormone from the sample, and correlating the obtained ratio level of conventionally determined iPTH and n-oxPTH to the risk of a myocardial infarction. In other words, the fraction of oxidized PTH molecules is a strong predictive marker for the oxidative stress suffered by said patient and for myocardial infarction. As patients suffering from CKD of high state have their iPTH levels conventionally determined about four times a year to monitor the risk of a chronic kidney disease-mineral and bone disorder (CKD-MBD) and renal osteodystrophy (ROD), a determination of n-oxPTH is a minor additional task which allows a control of the risks of coronary diseases and myocardial infarction, in addition to a better monitoring of the active PTH levels in accordance with the Kidney Disease Outcomes Quality Initiative (KDOQI) guidelines.

The method according to the invention can be used for determining the need of a patient of receiving a medication regulating the level of non-oxidized PTH in plasma or serum and for avoidance of oxidative stress and oxidation of the circulating PTH.

A most preferred embodiment lies in a method for determining the need of patient of receiving a medication reducing oxidative stress and in order to adjust the level of parathyroid hormone secretion into the circulation.

Further embodiments of the disclosed method and its advantages will become apparent from the detailed description, the examples and the scope of the claims. The examples and drawings are for illustration only. The scope of the invention will become apparent to the skilled person from the disclosure and scope of the claims.

### BRIEF DESCRIPTION OF THE DRAWINGS

In the figures:

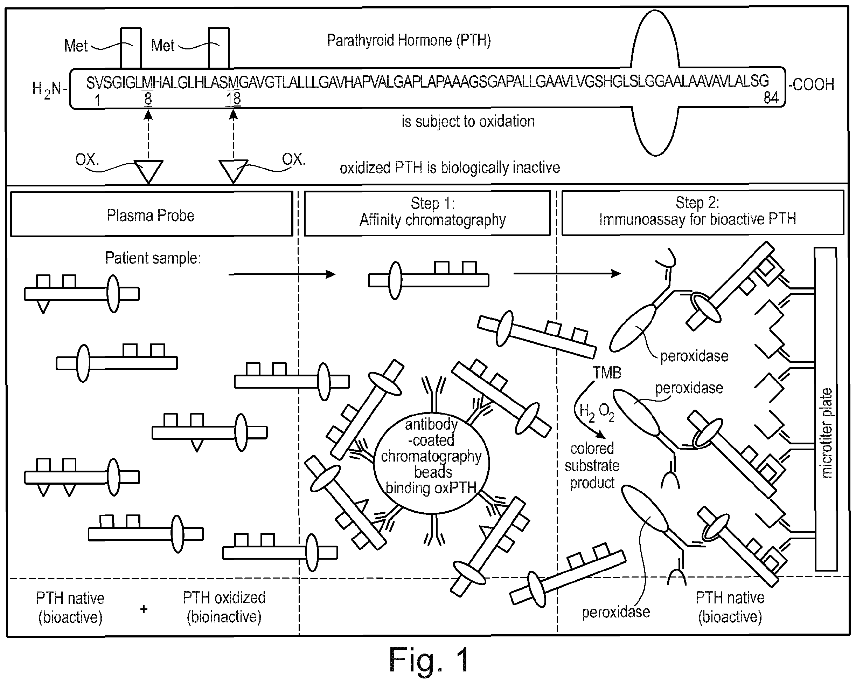

- **Fig. 1**: schematic representation of the measurement of parathyroid hormon (PTH) and the assay principle for measring non-oxidized PTH (NOX-PTH).

### DETAILED DESCRIPTION OF THE INVENTION

The invention is set out in the appended claims. Chronic kidney disease (CKD) affects 14.5% of the U.S. population with a host of biochemical and clinical abnormalities, including chronic kidney disease-mineral and bone disorder (CKD-MBD) and renal osteodystrophy (ROD). CKD-MBD refers to the clinical syndrome encompassing mineral, bone, and vascular calcification abnormalities that develop as complications of CKD, whereas ROD is used to describe the bone pathology of CKD. The laboratory diagnostic spectrum however needs expansion to include the risks of vascular complication and coronary diseases, and this applies generally to patients with high risk for developing coronary diseases and a myocardial infarction, which includes in particular patients with CKD. Clinical practice guidelines recommend that in patients with CKD serum PTH can be used to evaluate bone disease because markedly high or low values predict underlying bone turnover. There is no parallel biochemical monitoring of the risk of coronary disease available, but the present disclosure describes that a modified measurement as to the parathyroid hormone can be used therefore. Calcification of cardiac tissue can also affect the myocardium, the conduction system, and valves, and thus may cause adverse cardiovascular events, while myocardial infarction is a separate medical entity. Calcification of the aortic and/or mitral valve is quite common in adults receiving maintenance hemodialysis, as is myocardial infarction, with a prevalence of 2.5-5.0-fold greater as compared with non-hemodialysis patients with suspected and documented coronary artery disease. Cardiac calcification may also cause myocardial fibrosis and left ventricular dysfunction, which can culminate in heart failure, or when it involves the electrical conduction system, cardiac arrhythmias may result. Left ventricular hypertrophy has been identified as an independent risk factor for morbidity and mortality in the dialysis population.

Typical risk factors for myocardial infarction include high blood pressure, smoking, diabetes, obesity, high blood cholesterol, poor diet, and excessive alcohol intake, among others, including male sex, low levels of physical activity, a past family history. Risk factors for myocardial disease are often included in risk factor stratification scores, such as the Framingham risk score. At any given age, men are more at risk than women for the development of cardiovascular disease. Less common causes include stress-related causes such as job stress, which accounts for about 3% of cases, and chronic high stress levels. The blockage of a coronary artery caused by a rupture of an atherosclerotic plaque is usually the underlying mechanism but coronary artery spasms, significant emotional stress, and extreme cold are other mechanisms. A number of tests are useful to help with diagnosis, including electrocardiograms (ECGs), and coronary angiography. An ECG, which is a recording of the heart's electrical activity, may confirm an ST elevation MI (STEMI) if ST elevation is present. Commonly used blood tests include the cardiac protein troponin or the cardiac enzyme CK-MB since myocardial infarction occurs when there is cell death. The latter laboratory methods do not determine the risk of developing a myocardial infarction but serve for diagnosis of an existing myocardial tissue death (infarction) and detect damaged heart muscle (myocardium). Consequently, a biomarker as described is extremely useful for monitoring high risk CKD patients and persons suffering from various forms of stress.

Secondary hyperparathyroidism is associated with morbidity and mortality in patients with CKD stages 3-5. Observational studies consistently report an increased relative risk of death in CKD stage 5D patients who have measured PTH values at the extremes (less than 2 or greater than 9 times the upper normal limit of the assay). As described in EP 14711939, EP13158401, EP13708713, EP00989994 we have found that these patients tend to have normal hormonal PTH activity but large amounts of oxidized inactive PTH (oxPTH) in the circulation. Conventionally measured "intact PTH" (iPTH or intact PTH) does not distinguish between hormonal active PTH and oxidized inactive PTH (oxPTH). Progressive increases of iPTH values should be avoided and may point to increased levels of oxPTH and marked changes in iPTH levels should trigger a therapeutic response. The parallel determination of non-oxidized PTH (n-oxPTH) is needed to avoid an inadvertent therapy to the wrong if there are increased levels of iPTH. Monitoring PTH trends is important for the detection and treatment of CKD-MBD. Establishing target ranges is difficult because of the described methodological problems. Conventional 2^{nd} and 3^{rd} generation PTH immunoassays differ in their measurement and there is inter-assay and biological variability. Generally, high values of non-oxidized PTH (n-oxPTH) as determined using for example the PARATRIN Prolife^{®} serum sample preparation are combined a with reduced mortality whereas conventionally determined high intact PTH (iPTH) levels involve high mortality as those levels often contain a high proportion of oxidized inactive PTH molecules. Clinicians should futher be aware that certain populations, such as blacks with CKD, may have generally higher PTH levels. Bone turnover is also lower in blacks compared to whites. In patients with CKD or on hemodialysis, it is reasonable to base the frequency of PTH monitoring on the presence and magnitude of abnormalities and rate of progression of CKD. If PTH changes markedly in either direction within this range, a response is warranted to avoid progression to levels outside the range and a myocardial infarction.

At this stage, we would like to clarify again the various measurement methods and related problems. PTH is an 84 amino acid peptide that is cleaved both within the parathyroid gland and after secretion into the N-terminal, C-terminal, and mid-region fragments. There has been a progression of increasingly sensitive assays developed over the past few decades. The second generation assay, or "intact PTH" assay is the type of assay most widely used today while data demonstrates that the "intact assay" also detects oxidized inactive PTH.

Matrix-assisted laser desorption ionisation coupled with time-of-flight MS (MALDI-TOF MS) can be used used for peptide identification while sensitivity is still a hurdle. Zhang C-X et al describes in an article entitled Identification of carboxy-terminal peptide fragments of parathyroid hormone in human plasma at low-picomolar levels by mass spectrometry (Anal Chem 2006;78:1636-43) immunoextracted PTH fragments from healthy controls, patients with chronic renal disease, and from healthy post-menopausal women given recombinant human PTH(1-84). Extracted PTH fragments were analysed by MALDI-TOF MS following fractionation on a capillary LC system; and directly by nano-LC-TOF-MS. Four PTH fragments (PTH34-84, PTH37-84, PTH38-84 and PTH45-84) were identified as the major circulating fragments in samples from renal patients and from healthy volunteers given PTH1-84 (at estimated concentrations from 10 to 100 pmol/L). All of these fragments, with the exception of PTH(45-84), were also found in control samples. In 2010, Lopez MF et al published similar experiments in an article entitled Selected reaction monitoring-mass spectrometric immunoassay responsive to parathyroid hormone and related variants (Clin Chem 2010;56:281-90491. However, this time the serum PTH was enriched using an immunoaffinity process by having the antibody (polyclonal goat antihuman PTH39-84) immobilised on micro-columns embedded into pipette tips [mass spectrometric immunoassay (MSIA)]. In samples from patients with renal failure, PTH fragments observed were consistent with those observed by Zhang et al. previously. In addition, novel fragments with truncated N- and C-termini were also observed; these corresponded to PTH28-84, PTH48-84, PTH34-77, PTH37-77 and PTH38-77. Lopez et al. also went on to develop a quantitative assay for these PTH fragments using a triple quadrupole mass spectrometer (see 'Quantitative LC-MS/MS PTH assays'). However, in neither study were larger C-terminal PTH fragments observed, including the widely cited PTH7-84 variant to which the development of third-generation immunoassays can be largely credited. LC-MS analyses could not detect N-truncted PTH in serum of patients and unfortunately, assay kits from different manufacturers measure varying types and amounts of these circulating fragments, leading to inconsistent results. As oxidized PTH1-84 possesses almost identical properties as PTH7-84 on an HLPC column separation it seems likely that the oxidized inactive PTH has been mistaken as "N-truncated" PTH fragment. Addressing the need for 1-84 PTH standardization, The World Health Organization (WHO) Expert Committee on Biological Standardization (ECBS) reported a preparation of recombinant human 1-84 PTH to serve as the international standard, known as 95/646.23. The recommended target PTH levels of 150-300 pg/mL were arrived at between the late 1980s to the 1990s using the Nichols Allegro Intact PTH assay, which was the reference for PTH measurement until early 2006. But since then, inter-method variability has been shown to produce significantly different concentrations. In addition, most assays have been calibrated against synthetic 1-84 PTH from different sources. But even with that new standard, PTH concentrations varied in wide range and it was recommended that clinical laboratories inform clinicians of the actual assay method in use and report any change in methods, sample source (plasma or serum), and handling specifications to facilitate appropriate interpretation of biochemical data. The use of correction factors to overcome the inter-method variability of PTH measurement cannot represent a solution, while still considered a pragmatic approach, because PTH oxidation differs among patients and with time. The conventional 2^{nd} and 3^{rd} generation PTH assays do not take into account the variable proportions of non-oxidized active PTH versus oxidized inactive PTH.

We have found that 2^{nd} and 3^{rd} generation PTH assays such the Nichols Allegro intact PTH assay, Bayer PTH Advia Centaur^{™}, Scantibodies Total intact PTH, and Roche Elecsys^{™} could produce consistent PTH results provided of a pretreatment of the sample using the beads binding and removing oxidized PTH molecules contained in the serum sample. In other words, a double measurement will allow the determination of the individual difference or a ratio of n-oxPTH and iPTH. The result can then be used to determine additionally the relative risk of a myocardial infarction. Thus, oxPTH, n-oxPTH and conventional iPTH can be used as a predictive biomarker for myocardial infarction, if the amount of oxidized PTH or the amount of the non-oxidized PTH is determined in the sample. The resulting biomarker will then describe the risk of a coronary disease or myocardial infarction or a severe cardiovascular event as a diagnostic endpoint.

### EXAMPLE

1048 Patients undergoing angiography for established or suspected stable CAD were followed up for 6.3 ± 1.7 years. See Table 1 below for baseline characteristics. The primary study end point, cardiovascular events, was a composite of coronary death, ischemic stroke, myocardial infarction, CABG, PCI, or carotidlperipheral revascularization. N-oxPTH was determined using the method described in Fig. 1, which is available commercially (immundiagnostik AG, Bensheim, DE - Paratrin Prolife^{®}). The baseline characteristics of the patient have been summarized in Table 1.

**TABLE 1**

| | | | |
|---|---|---|---|
| At baseline | Male | Female | P value |
| | | | |
| N | 677 | 371 | |
| Diabetes ADA (n [%]) | 192 (28.4) | 92 (24.8) | N5 |
| Smoking history (n [%]) | 485 (71.6) | 137 (36.9) | < 0.001 |
| CAD (n [%]) | 604 (89.2) | 247 (66.6) | < 0.001 |
| Cerebral insult (n [%]) | 30 (4.5) | 22 (6.0) | N5 |
| PAVK (n [%]) | 79 (11.8) | 28 (7.7) | 0.052 |
| Age | 63.7 (11.1) | 66.7 (9.6) | < 0.001 |
| BNP, pg/ml | 42.7 (3.45.4) | 36.1 (72.8) | NS |
| CRP, mg/l | 0.42 (0.70) | 0.44 (0.74) | NS |
| Tnl, pg/ml | 18.0 (74.1) | 64.6 (706.1) | NS |
| TG, mg/dl | 143.1 (92.2) | 131.7 (80.1) | 0.047 |
| LDL, mg/dl | 124.4 (40.3) | 132.5 (421.4) | 0.002 |
| HDL, mg/dl | 52.5 (3.4.3) | 63.6(18.3) | < 0.001 |
| Vitamin D, nmol/l | 43.8 (23.8) | 38.8 (20.9) | 0.001 |
| N-oxPTH, pg/ml | 8.9 (19.3) | 11.3 (21.6) | 0.072 |
| PTH, pg/ml | 41.8 (30.0) | 45.8 (30.8) | 0.050 |
| FGF-23, pmol/l | 1.1 (2.2) | 1.3 (2.8) | NS |
| Klotho, ng/ml | 13.3 (10.8) | 13.9 (11.1) | NS |
| GFR CKD-EPI, ml/min/1.73 m2 | 87.3 (23.2) | 80.5 (20.6) | < 0.001 |

Table 2 (overleaf) shows the preliminary results of Cox analyses. The Cox model has been adjusted for age and sex, HR (Hazard Risk) per SD (standard deviation) for metric variables and in decremental order of p value.

Note that for stroke, CABG, and revascularization (not shown), neither n-oxPTH nor PTH showed significant impact. However, for myocardial infarction N-oxPTH proved to be useful as a predictor and *specific* biomarker for **myocardial infarction,** in combination with an 2^{nd} or 3^{rd} generation iPTH measurement. The combined results also allow a better monitoring of those patients which are in need of a treatment requiring adjustment of the PTH levels. Cardivascular events and cardiovascular mortality include all type of cardiovascular events, including stroke. Heart includes all type of events, including angiography, stents, etc. This comparison emphasized that NOX-PTH is a strong biomarker and can be used for determining a risk factor for myocardial infarction which has not previously been noted. This *in vitro* serum marker is further unexpected as there is no apparent physiological correlation or mechanism between PTH in serum and myocardial infarction. And even a better marker than the established BNP and NT-BNP. As PTH is rapidly degraded with minutes after secretion this may further indicate that any one of the PTH fragments must have biological activity which has not been described nor noted. It is further noteworthy that PTH, when determined conventionally, is as such a less potent predictor for myocardial infarction.

This analysis is further the first report that of cardio-vascular risk prediction on the basis of a PTH measurements. Non-oxidized (n-ox)PTH is further superior to conventional iPTH measurement also in patients without end-stage renal disease or not subject to dialysis treatment. A very stronger predictor of myocardial infarction is the ratio of ox-PTH / n-oxPTH (level of oxidized PTH / non-oxidized PTH) or the absolute amount of oxPTH which indicates that the risk of myocardial infarction is strongly associated to the oxidative stress suffered by the patient. It is well know that CKD suffer from oxidative stress due to inflammation, and even worse when being subjected to hemodialysis. It is very likely that their increased risk for myocardial infarction can be monitored using the disclosed biomarker for myocardial infarction.

**TABLE 2**

| Cardiovascular Events | | | Cardiovascular Mortality | | | Myocardial Infarction | | | Heart | | |
|---|---|---|---|---|---|---|---|---|---|---|---|
| Variable HR | | P value | Variable | HR | P value | Variable | | P value | Variable | HR | P value |
| CAD | 3.0 (1.9-4.8) | <0.0001 | HbAlc | 1.4 (1.2-1.7) | 0.0001 | **N-oxPTH** | **1.2(1.1-1.3)** | **0.0001** | CAD | 3.2 (1.9-5.3) | <0.0001 |
| PAD | 2.3 (1.7-3.1) | <0.0001 | Diabetes | 2.3 (1.5-3.6) | 0-0002 | PTH | 1.3(1.1-1.5) | 0.0003 | CRP | 1.2 (1.1-1.3) | <0.0001 |
| CRP | 1.2 (1.1-1.3) | <0.0001 | GFR | 0.6 (0.5-0.8) | 0-0002 | GFR | 0.6(0.5-0.8) | 0.0005 | HDL | 0.7 (0.6-0.8) | <0.0001 |
| HDL | 0.7 (0.6-0.8) | <0.0001 | HDL | 0.7 (0.5-0.9) | 0-002 | PAD | 2.7(1.5-4.9) | 0.0006 | PAD | 1.9 (1.3-2.6) | 0-0003 |
| HbAlc | 1.2 (1.1-1.3) | 0.0007 | VitD | 0.6 (0.5-0.9) | 0-003 | HDL | 0.6(0.5-0.9) | 0.004 | HbAlc | 1.2 (1.1-1.3) | 0-0007 |
| GFR | 0.8 (0.7-0.9) | 0.001 | FGF-23 | 1.2 (1.1-1.4) | 0-004 | Smoking | 1.8(1.1-3.2) | 0.031 | ***N-oxPTH*** | 1.1 (1.0-1.2) | 0.004 |
| Diabetes | 1.4 (1.1-1.8) | 0.004 | CRP | 1.2 (1.1-1.4) | 0-005 | CRP | 1.2(1.0-1.3) | 0.038 | GFR | 0.8 (0.7-0.9) | 0.005 |
| TG | 1.2 (1.0-1.3) | 0.005 | BNP | 1.1 (1.0-1.2) | 0-009 | VitD | 0.7(0.6-1.0) | 0.056 | VitD | 0.8 (0.7-0.9) | 0.010 |
| BNP | 1.1 (1.0-1.2) | 0.005 | PAD | 1.9 (1.1-3.4) | 0-019 | Klotho | 0.9(0.7-1.2) | 0.579 | Diabetes | 1.4 (1.1-1.8) | 0.010 |
| VitD | 0.8 (0.7-0.9) | 0.008 | ***N-oxPTH*** | 1.1 (0.9-1.2) | 0.320 | FGF-23 | 1.0(0.6-1.4) | 0.827 | TG | 1.2 (1.0-13) | 0.018 |
| Smoking | 1.4 (1.1-1.9) | 0.010 | Klotho | 1.1 (0.8-1.3) | 0.600 | | | | ***PTH*** | 1.1 (1.0-1.3) | 0.042 |
| ***N-oxPTH*** | 1.1 (1.0-1.2) | 0.018 | ***PTH*** | 1.0 (0.8-1.3) | 0.824 | | | | FGF-23 | 1.0 (0.6-1.4) | 0.052 |
| Tnl | 1.1. (1.0-1.2) | 0.030 | | | | | | | Klotho | 1.0 (0.8-1.1) | 0.617 |
| PTH | 1.1 (0.9-1.2) | 0.096 | | | | | | | | | |
| FGF-23 | 1.1 (0.9-1.2) | 0.115 | | | | | | | | | |
| Klotho | 0.9 (0.8-1.1) | 0.335 | | | | | | | | | |

## Claims

1. Method of determining the health risk for myocardial infarction in patients subjected to coronary angiography, wherein the concentration of the parathyroid hormone is determined in vitro in serum or plasma sample of said patient **characterized in that** the serum or plasma sample is first contacted with beads specifically binding oxidized parathyroid hormone and correlating the determined level of oxidized parathyroid hormone molecules to the health risk for myocardial infarction, wherein the concentration of the parathyroid hormone is determined in vitro in a first sample of serum or plasma of said patient and in a second sample of serum or plasma of said patient after contacting said sample with beads binding oxidized parathyroid hormone and correlating the determined levels of parathyroid hormone in said first and second sample to the risk of developing a myocardial infarction by said patient.

2. Method as claimed in claim 1, wherein the amount of parathyroid hormone is determined using a second or third generation PTH immunoassay before and after removing the oxidized parathyroid hormone from said sample.

3. Method as claimed in claim 1, comprising a correlation of the ratio of determined PTH in serum or plasma and n-oxPTH in serum or plasma to the risk of developing a myocardial infarction.

## Patentansprüche

1. Verfahren zur Bestimmung des Gesundheitsrisikos für Herzinfarkt bei Patienten, die einer Koronarangiographie unterzogen werden, wobei die Konzentration des Parathormons in vitro in einer Serum- oder Plasmaprobe des Patienten bestimmt wird, **dadurch gekennzeichnet, dass** die Serumprobe zunächst mit Beads in Kontakt gebracht wird, die spezifisch oxidiertes Parathormon binden, und dass der ermittelte Gehalt an oxidierten Parathormonmolekülen mit dem Gesundheitsrisiko für Herzinfarkt korreliert wird, dass die Konzentration des Parathormons *in vitro* in einer ersten Serum- oder Plasmaprobe des Patienten und in einer zweiten Serum- oder Plasmaprobe des Patienten bestimmt wird, nachdem die Probe mit Beads in Kontakt gebracht wurde, die oxidiertes Parathormon binden, und dass die bestimmten Parathormon-Spiegel in der ersten und zweiten Probe mit dem Risiko des Patienten, einen Myokardinfarkt zu entwickeln, korreliert werden..

2. Verfahren nach Anspruch 1, wobei die Menge an Parathormon unter Verwendung eines PTH-Immunoassays der zweiten oder dritten Generation vor und nach Entfernung des oxidierten Parathormons aus der Probe bestimmt wird.

3. Verfahren nach Anspruch 1, umfassend eine Korrelation des Verhältnisses von bestimmtem PTH im Serum oder Plasma und n-oxPTH im Serum oder Plasma mit dem Risiko, einen Herzinfarkt zu erleiden.

## Revendications

1. Méthode de détermination du risque d'infarctus du myocarde chez des patients soumis à une coronarographie, dans laquelle la concentration de l'hormone parathyroïdienne est déterminée *in vitro* dans un échantillon de sérum ou de plasma dudit patient, **caractérisée par le fait que** l'échantillon de sérum est d'abord mis en contact avec des billes liant spécifiquement l'hormone parathyroïdienne oxydée et par la corrélation du niveau déterminé de molécules d'hormone parathyroïdienne oxydée au risque d'infarctus du myocarde, dans lequel la concentration de l'hormone parathyroïdienne est déterminée *in vitro* dans un premier échantillon de sérum ou de plasma dudit patient et dans un second échantillon de sérum ou de plasma dudit patient après avoir mis en contact ledit échantillon avec des billes liant l'hormone parathyroïdienne oxydée et en corrélant les niveaux déterminés de l'hormone parathyroïdienne dans ledit premier et second échantillon au risque de développer un infarctus du myocarde chez ledit patient.

2. Méthode selon la revendication 1, dans laquelle la quantité d'hormone parathyroïdienne est déterminée à l'aide d'un dosage immunologique de la PTH de deuxième ou troisième génération avant et après l'élimination de l'hormone parathyroïdienne oxydée de l'échantillon.

3. Méthode selon la revendication 1, comprenant une corrélation entre le rapport entre la PTH déterminée dans le sérum ou le plasma et la n-oxPTH dans le sérum ou le plasma et le risque de développer un infarctus du myocarde.
